(19) Europäisches Patentamt
European Patent Office
Office européen des brevets

(11) Publication number: **0 082 607 A1**

(12) **EUROPEAN PATENT APPLICATION**

(21) Application number: 82306307.8

(22) Date of filing: 26.11.82

(51) Int. Cl.³: C 07 D 251/16
C 07 D 251/22, C 07 D 251/44
C 07 D 251/46, C 07 D 251/50
C 07 D 251/52, C 07 D 239/42
C 07 D 239/46, C 07 D 239/48
C 07 D 239/52, C 07 D 491/04
//(C07D491/04, 311/00, 239/00),
(C07D491/04, 307/00, 239/00)

(30) Priority: 30.11.81 US 325800

(43) Date of publication of application: 29.06.83 Bulletin 83/26

(84) Designated Contracting States: DE FR GB IT

(71) Applicant: PPG INDUSTRIES, INC.
One Gateway Center
Pittsburgh Pennsylvania 15222(US)

(72) Inventor: Richter, Sidney Bernard
2601 Sand Run Parkway
Fairlawn Ohio 44313(US)

(72) Inventor: Krass, Dennis Keith
507 Dan Avenue
Canal Fulton Ohio 44614(US)

(74) Representative: Shipton, Gordon Owen et al,
W.P. THOMPSON & CO. Coopers Building Church Street
Liverpool L1 3AB(GB)

(54) **Herbicidally active 1-(2-(substituted sulfonyl aminocarbonyl)-phenyl)-3-heterocyclyl urea derivatives.**

(57) Compounds represented by the formula:

I. 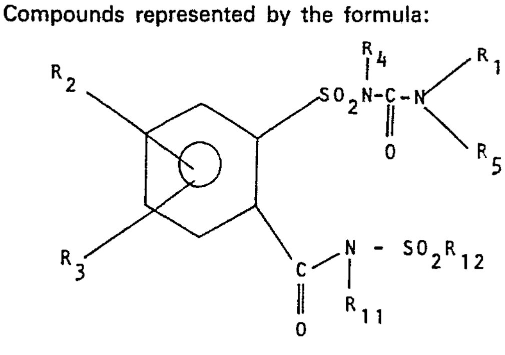

II 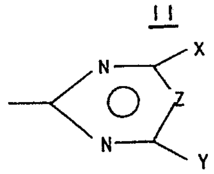

III 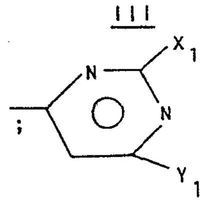

IV 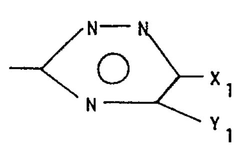

V 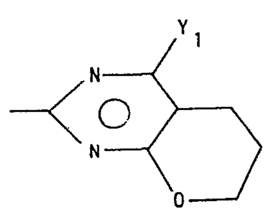

wherein,
R¹ is a group structurally represented by the Formulae II through VII:

./...

EP 0 082 607 A1

VI

;

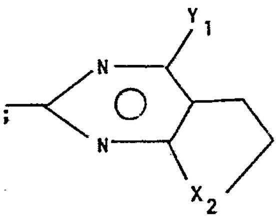

VIII

,

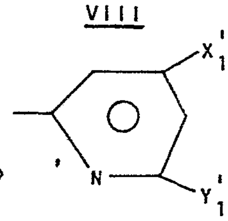

wherein

X is H, Cl, Me, MeO, EtO or $MeO(CH_2)_2O$;

Z is CH, N, CF, CCl, CBr, CMe, COMe, CEt or $C-(CH_2)_2Cl$;

Y is H, Cl, $C_1$ to $C_4$ alkyl which may be substituted by MeO, EtO, CN, $CO_2Me$, $CO_2Et$, or 1 to 3 F, Cl or Br; $C_3$ to $C_4$ alkenyl; $CH_2C{\equiv}CR_6-$; $-A(CH_2)_m-A_1-(C_1$ to $C_3$ alkyl); $-A-CH_2-COL$; $-A-CHMe-COL$; $-A(CH_2-COL$; $-SCN$; $N_3$, $NR_7R_8$, $OR_9$ or $SR_{10}$;

$R_6$ is H, Me or $CH_2Cl$;

m is 2 or 3;

A is O or S;

$A_1$ is O, S or $SO_2$;

L is OH, $NH_2$, $-NMe-OMe$, mono- or di-($C_1$ to $C_4$ alkyl) amino or $C_1$ to $C_6$ alkoxy;

$R_7$ is H or Me;

$R_8$ is H, MeO, $C_1$ to $C_4$ alkyl, $C_3$ to $C_6$ cycloalkyl, $C_1$ to $C_4$ alkyl substituted by CN, $CO_2Me$ or $CO_2Et$, $C_3$ to $C_4$ alkenyl, or $C_2$ to $C_3$ alkyl substituted by MeO or EtO; or $R_7$ and $R_8$ combine to form $(CH_2)_4$ or $(CH_2)_2-O-(CH_2)_2$;

$R_9$ is $C_1$ to $C_4$ alkyl; $C_2$ to $C_4$ alkyl substituted by 1 to 3 F, Cl or Br; $C_1$ to $C_4$ alkyl substituted by CN, $C_3$ to $C_4$ alkenyl, $CR_6{=}C-CH_2-$cyclopropylmethyl or 3-tetrahydrofuryl;

$R_{10}$ is $C_1$ to $C_4$ alkyl, or $C_1$ to $C_2$ alkyl substituted by CN, allyl or propargyl;

$X_1$ is H, Cl, MeO, EtO or Me;

$Y_1$ is H, MeO or Me;

$X_2$ is O or $CH_2$;

$X'_1$ and $Y'_1$ are Me or MeO;

$R_2$ is H, F, Cl, Br, $C_1$ to $C_4$ alkyl, $NO_2$, $CH_3SO_2-$, MeO, MeS, $CF_3$, $(CH_3)_2N-$, $NH_2$ or CN;

$R_3$ is H, F, Cl, Br or $CH_3$;

$R_4$ is H or $CH_3$;

$R_5$ is H, $CH_3$ or $OCH_3$;

$R_{11}$ is H or $C_1$ to $C_4$ alkyl; and

$R_{12}$ is (a) phenyl, pyridyl or thionyl, each optionally substituted by halogen, alkyl or $NO_2$;

(b) $C_1$ to $C_4$ alkenyl or alkynyl; or

(c) $C_1$ to $C_4$ alkyl optionally substituted by Cl, Br, I, $CO_2H$, $C_2$ to $C_5$ alkoxy carbonyl, $C_2$ to $C_5$ alkanoyl, mono- or di-($C_1$ to $C_4$ alkyl) carbamoyl, $C_1$ to $C_4$ alkoxy, $C_1$ to $C_4$ alkylthio, sulphinyl or sulphonyl, $C_2$ to $C_5$ alkanoyloxy, $C_2$ to $C_5$ alkanoylamino or cyano.

# DESCRIPTION

## HERBICIDALLY ACTIVE 1-[2-(SUBSTITUTED SULFONYL AMINOCARBONYL)-PHENYL]-3-HETEROCYCLYL UREA DERIVATIVES

This invention relates to certain 1-[2-(substituted sulfonyl aminocarbonyl)-phenyl]-3-heterocyclyl urea derivatives structurally represented by the Formula I:

I. 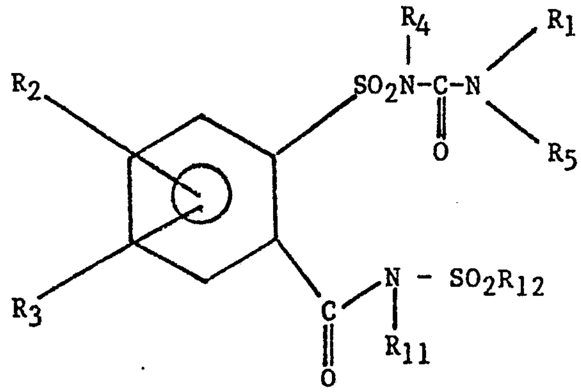

wherein,

$R^1$ is a group structurally represented by the Formulae II through VII:

II

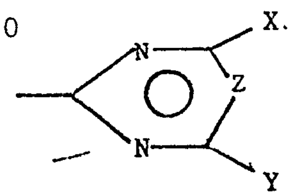

;

III

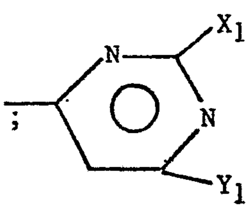

;

IV

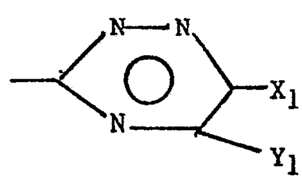

,

V

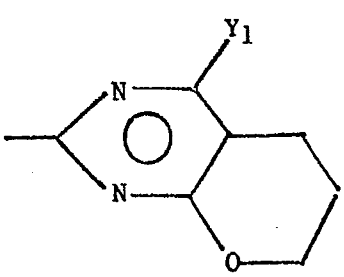

;

VI

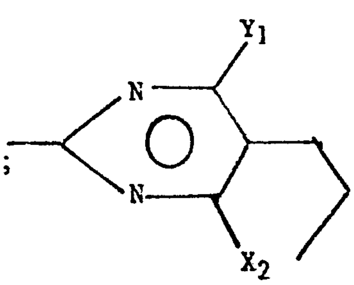

;

VII

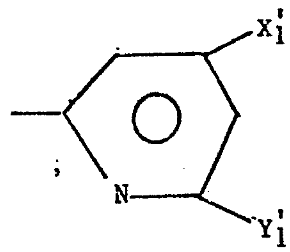

wherein

X is H, Cl, Me, MeO, EtO or $MeO(CH_2)_2O$,

Z is CH, N, CF, CCl, CBr, CMe, COMe, CEt or $C-(CH_2)_2Cl$;

Y is H, Cl, $C_1$ to $C_4$ alkyl which may be substituted by MeO, EtO, CN, $CO_2Me$, $CO_2Et$, or 1 to 3 F, Cl or Br; $C_3$ to $C_4$ alkenyl, $CH_2C{\equiv}CR_6-$, $-A(CH_2)_m-A_1-(C_1$ to $C_3$ alkyl); $-A-CH_2-COL$; $-A-CHMe-COL$; $- A(CH_2-COL$; $-SCN$; $N_3$, $NR_7R_8$, $OR_9$ or $SR_{10}$;

$R_6$ is H, Me or $CH_2Cl$;

m is 2 or 3;

A is O or S;

$A_1$ is O, S or $SO_2$;

L is OH, $NH_2$, $-NMe-OMe$, mono- or di-($C_1$ to $C_4$ alkyl) amino or $C_1$ to $C_6$ alkoxy;

$R_7$ is H or Me;

$R_8$ is H, MeO, $C_1$ to $C_4$ alkyl, $C_3$ to $C_6$ cycloalkyl, $C_1$ to $C_4$ alkyl substituted by CN, $CO_2Me$ or $CO_2Et$, $C_3$ to $C_4$ alkenyl, or $C_2$ to $C_3$ alkyl substituted by MeO or EtO; or $R_7$ and $R_8$ combine to form $(CH_2)_4$ or $(CH_2)_2-O-(CH_2)_2$;

$R_9$ is $C_1$ to $C_4$ alkyl; $C_2$ to $C_4$ alkyl substituted by 1 to 3 F, Cl or Br, $C_1$ to $C_4$ alkyl substituted by CN, $C_3$ to $C_4$ alkenyl, $CR_6{=}C-CH_2$-cyclopropylmethyl or 3-tetrahydrofuryl;

$R_{10}$ is $C_1$ to $C_4$ alkyl, or $C_1$ to $C_2$ alkyl substituted by CN, allyl or propargyl;

$X_1$ is H, Cl, MeO, EtO or Me;

$Y_1$ is H, MeO or Me,

$X_2$ is O or $CH_2$;

$X_1'$ and $Y_1'$ are Me or MeO;

$R_2$ is H, F, Cl, Br, $C_1$ to $C_4$ alkyl, $NO_2$, $CH_3SO_2-$, MeO, MeS, $CF_3$, $(CH_3)_2N-$, $NH_2$ or CN,

$R_3$ is H, F, Cl, Br or $CH_3$;

$R_4$ is H or $CH_3$;

$R_5$ is H, $CH_3$ or $OCH_3$,

$R_{11}$ is H or $C_1$ to $C_4$ alkyl; and

$R_{12}$ is (a) phenyl, pyridyl or thionyl, each optionally substituted by halogen, alkyl or $NO_2$;

(b) $C_1$ to $C_4$ alkenyl or alkynyl; or

(c) $C_1$ to $C_4$ alkyl optionally substituted by Cl, Br, I, $CO_2H$, $C_2$ to $C_5$ alkoxy carbonyl, $C_2$ to $C_5$ alkanoyl, mono- or di-($C_1$ to $C_4$ alkyl) carbamoyl, $C_1$ to $C_4$ alkoxy, $C_1$ to $C_4$ alkylthio, sulphinyl or sulphonyl, $C_2$ to $C_5$ alkanoyloxy, $C_2$ to $C_5$ alkanoylamino or cyano.

Compounds of this invention embodied in the Formula I are very active broad spectrum herbicides and are effective in regulating growth of a wide variety of undesirable plants, i.e., weeds, when applied, in herbicidally effective amount, to the growth medium prior to emergence of the weeds or to the weeds subsequent to emergence from the growth medium. The term "herbicidally effective amount" is that amount of compound or mixture of compounds of this invention required to so injure or damage weeds such that the weeds are incapable of recovering following application. The quantity of compound or mixture of compounds of this invention applied in order to exhibit a satisfactory herbicidal effect may vary over a wide range and depends on a variety of factors, such as, for example, hardiness of a particular weed species, extent of weed infestation, climatic conditions, soil conditions, method of application and the like. Typically, as little as 0.1 or less pound per acre of compound or mixture of compounds of this invention would be expected to provide satisfactory weed control, although in some instances application rates in excess of one pound per acre might be required. Of course, the efficacy of a particular compound against a particular weed species may readily be determined by routine laboratory or field testing in a manner well known to the art.

A compound or compounds of this invention may, of course, be used as such or in formulation with agronomically acceptable

adjuvants, inert carriers, other herbicides, or other commonly used agricultural compounds, for example, insecticides, fungicides, stabilizers, safeners, fertilizers or the like. The compounds of this invention alone or in formulation with other agronomically used materials are typically applied in the form of dusts, granules, wettable powders, solutions, suspensions, aerosols, emulsions, dispersions or the like in a manner well known to the art. When formulated with other typically used agronomically acceptable materials, the amount of compound or compounds of this invention may vary over a wide range, for example, from about 0.05 to about 95 per cent by weight on weight of formulation. Typically, such formulations would contain from about 5 to about 75 percent by weight of compound or compounds of this invention.

A compound or compounds of this invention are effective in controlling a variety of common broadleaved and grassy weeds at application rates of only a few grams per acre either pre- or postemergent. Exemplary of weeds that may be effectively controlled by the application of compounds of this invention are barnyard grass (Echinochloa crusgalli), crabgrass (Digitaria sauguinalis), coffeeweed (Daubentonia punices), jimsonweed (Datura stamonium), johnsongrass (Sorghum halepense), tall morningglory (Ipomoea purpurea), wild mustard (Brassica caber), teaweed (Sida Spinosa), velvetleaf (Abutilon Theophrasti), wild oats (Avena fatua), yellow foxtail (Setaria glauca), yellow nutsedge (Cyperus esculentus) and the like.

Compounds of this invention may be prepared for example by reacting a compound of the Formula VIII:

VIII.

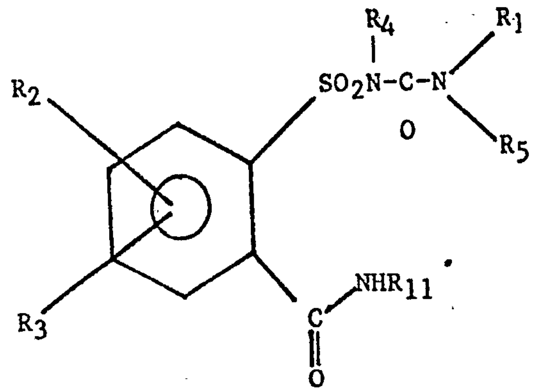

wherein $R_1$, $R_2$, $R_3$, $R_4$, $R_5$ and $R_{11}$ are as defined with reference to the compounds of Formula I, with at least an equimolar amount of sulfonyl halide of the formula, $R_{12}SO_2$-Hal, wherein Hal is halogen, preferably bromine or chlorine and $R_{12}$ is as defined with reference to the compounds of Formula I. The reaction between the Formula VIII compound and the sulfonyl halide is typically conducted in an inert non-protic solvent in the presence of an acid acceptor at a temperature ranging from ambient to reflux and the reaction mixture worked-up in known fashion to isolate the compound of this invention.

Alternatively, compounds of this invention may be prepared for example by reacting a compound of the Formula IX:

IX.

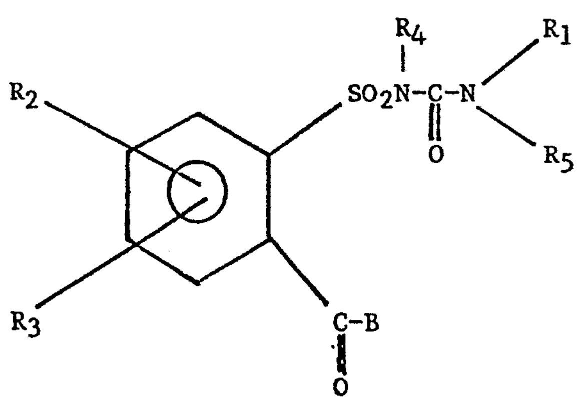

wherein $R_1$, $R_2$, $R_3$, $R_4$ and $R_5$ are as defined with reference to the compounds of Formula I and B is halogen, hydroxy, or alkoxy with at least an equimolar amount of a sulfonylamine of the formula

$NH(R_{11})SO_2R_{12}$ wherein $R_{11}$ and $R_{12}$ are as defined with reference to the compounds of Formula I. The reaction between the compound of Formula IX and the sulfonylamine is typically conducted in an inert non-protic solvent and in the presence of an acid acceptor solvent at a temperature ranging from ambient to reflux and the reaction mixture worked-up in known fashion to isolate a compound of the invention.

Regarding starting materials for the above described preparations, the sulfonyl halide and sulfonyl amine can be obtained from commerical sources or synthesized by known techniques, whereas the preparation of compounds of the Formulae VIII and IX is described in European Patent Application Publication No. 0030138, the teachings of which are incorporated by reference herein as regards the mode of preparation of said compounds.

Although the invention has been described in some detail with reference to certain embodiments thereof, it is to be understood that it is not intended to be so limited, since many variations may be made therein by those skilled in the art without departing from the spirit and scope thereof, except as defined in the appended claims.

..................................................

CLAIMS

1. A compound represented by the formula:

I. 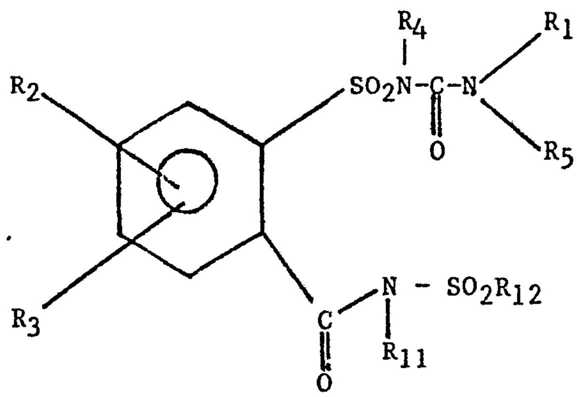

wherein,

$R^1$ is a group structurally represented by the Formulae II through VII:

II

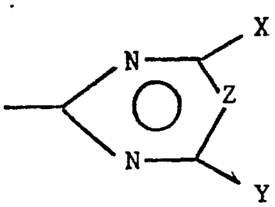

III

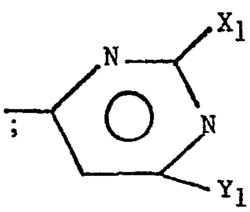

;

IV

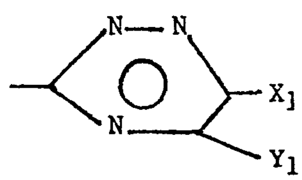

;

,

V

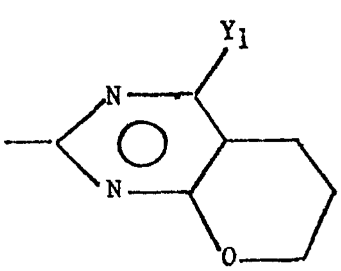

VI

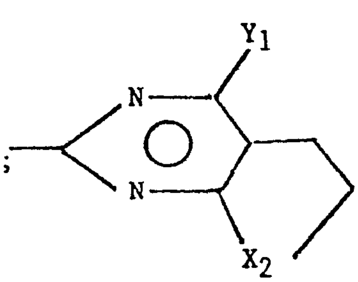

;

VII

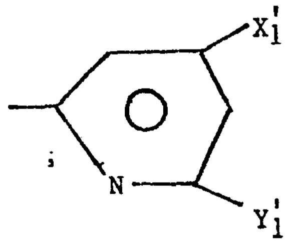

;

wherein

X is H, Cl, Me, MeO, EtO or $MeO(CH_2)_2O$;

Z is CH, N, CF, CCl, CBr, CMe, COMe, CEt or $C-(CH_2)_2Cl$;

Y is H, Cl, $C_1$ to $C_4$ alkyl which may be substituted by MeO, EtO, CN, $CO_2Me$, $CO_2Et$, or 1 to 3 F, Cl or Br; $C_3$ to $C_4$ alkenyl; $CH_2C{\equiv}CR_6$-; $-A(CH_2)_m-A_1-(C_1$ to $C_3$ alkyl); $-A-CH_2-COL$; $-A-CHMe-COL$; - $A(CH_2-COL$; -SCN; $N_3$, $NR_7R_8$, $OR_9$ or $SR_{10}$;

$R_6$ is H, Me or $CH_2Cl$;

m is 2 or 3;

A is O or S;

$A_1$ is O, S or $SO_2$;

L is OH, $NH_2$, -NMe-OMe, mono- or di-($C_1$ to $C_4$ alkyl) amino or $C_1$ to $C_6$ alkoxy;

$R_7$ is H or Me;

$R_8$ is H, MeO, $C_1$ to $C_4$ alkyl, $C_3$ to $C_6$ cycloalkyl, $C_1$ to $C_4$ alkyl substituted by CN, $CO_2Me$ or $CO_2Et$, $C_3$ to $C_4$ alkenyl, or $C_2$ to $C_3$ alkyl substituted by MeO or EtO; or $R_7$ and $R_8$ combine to form $(CH_2)_4$ or $(CH_2)_2-O-(CH_2)_2$;

$R_9$ is $C_1$ to $C_4$ alkyl; $C_2$ to $C_4$ alkyl substituted by 1 to 3 F, Cl or Br; $C_1$ to $C_4$ alkyl substituted by CN, $C_3$ to $C_4$ alkenyl, $CR_6{=}C-CH_2$-cyclopropylmethyl or 3-tetrahydrofuryl;

$R_{10}$ is $C_1$ to $C_4$ alkyl, or $C_1$ to $C_2$ alkyl substituted by CN, allyl or propargyl;

$X_1$ is H, Cl, MeO, EtO or Me;

$Y_1$ is H, MeO or Me;

$X_2$ is O or $CH_2$;

$X_1'$ and $Y_1'$ are Me or MeO;

$R_2$ is H, F, Cl, Br, $C_1$ to $C_4$ alkyl, $NO_2$, $CH_3SO_2$-, MeO, MeS, $CF_3$, $(CH_3)_2N$-, $NH_2$ or CN;

$R_3$ is H, F, Cl, Br or $CH_3$;

$R_4$ is H or $CH_3$;

$R_5$ is H, $CH_3$ or $OCH_3$;

$R_{11}$ is H or $C_1$ to $C_4$ alkyl; and

$R_{12}$ is (a) phenyl, pyridyl or thionyl, each optionally substituted by halogen, alkyl or $NO_2$;

(b) $C_1$ to $C_4$ alkenyl or alkynyl; or

(c) $C_1$ to $C_4$ alkyl optionally substituted by Cl, Br, I, $CO_2H$, $C_2$ to $C_5$ alkoxy carbonyl, $C_2$ to $C_5$ alkanoyl, mono- or di-($C_1$ to $C_4$ alkyl) carbamoyl, $C_1$ to $C_4$ alkoxy, $C_1$ to $C_4$ alkylthio, sulphinyl or sulphonyl, $C_2$ to $C_5$ alkanoyloxy, $C_2$ to $C_5$ alkanoylamino or cyano.

.....................................................................

European Patent Office

which under Rule 45 of the European Patent Convention shall be considered, for the purpose of subsequent proceedings, as the European search report

0082607

EP 82 30 6307

| The Search Division considers that the present European patent application does not comply with the provisions of the European Patent Convention to such an extent that it is not possible to carry out a meaningful search into the state of the art on the basis of all claims<br>Reason | CLASSIFICATION OF THE APPLICATION (Int Cl 3) |
|---|---|
| Without prejudging any decision of the examing division, the subject matter of the application seems not to be patentable, see articles 52(2) c), 83 and 84 and rules 27 (1) f and 29(1) of the EPC; therefore it has not been searched (rule 45 EPC) | C 07 D 251/16<br>251/22<br>251/44<br>251/46<br>251/50<br>251/52<br>239/42<br>239/46<br>239/48<br>239/52<br>239/70<br>213/75<br>253/06<br>491/04<br>401/12<br>409/12<br>A 01 N 47/36//<br>(C 07 D 491/04<br>311/00<br>239/00)<br>(C 07 D 491/04<br>307/00<br>239/00) |

| Place of search | Date of completion of the search | Examiner |
|---|---|---|
| The Hague | 12-01-1983 | VAN BIJLEN |